# EUROPÄISCHE PATENTSCHRIFT

(11) **EP 1 033 949 B1**
(45) Veröffentlichungstag und Bekanntmachung des Hinweises auf die Patenterteilung: **25.04.2007**
(21) Anmeldenummer: 98916857.0
(22) Anmeldetag: 04.03.1998
(51) Int. Cl.: A61F 2/46, B01F 13/00

(54) **BEHÄLTNIS ZUM MISCHEN, AB- UND UMFÜLLEN EINES STOFFGEMISCHES**
CONTAINER FOR MIXING, FILLING AND REFILLING A MIXTURE OF SUBSTANCES
CONTENANT PERMETTANT DE MELANGER, D'EMPAQUETER ET DE TRANSVASER UNE COMPOSITION

(30) Priorität: 06.03.1997 DE 19709036
(43) Veröffentlichungstag der Anmeldung: 13.09.2000
(73) Patentinhaber: Thurgauer Kantonalbank, 8570 Weinfelden (CH)
(72) Erfinder: Draenert, Klaus, 81545 München (DE)
(74) Vertreter: Rentsch & Partner
(86) Internationale Anmeldenummer: PCT/DE1998/000644
(87) Internationale Veröffentlichungsnummer: WO 1998/038950

(56) Entgegenhaltungen:
- WO-A-87/05492
- WO-A-94/26403
- US-A- 4 408 690
- PATENT ABSTRACTS OF JAPAN vol. 013, no. 039 (C-563), 27.Januar 1989 & JP 63 236556 A (OSAKA EYAZOOLE KOGYO KK), 3.Oktober 1988,

## Beschreibung

Die Erfindung betrifft eine Vorrichtung zum Abpacken von mindestens einer Komponente eines Gemischansatzes aus mindestens zwei Komponenten. Insbesondere betrifft die Erfindung eine Vorrichtung zum Mischen von Knochenzement auf PMMA-Basis in einem evakuierbaren Mischbecher.

Unter "Mischen" versteht man allgemein das Einbringen von Teilen eines Stoffes zwischen die Teile anderer Stoffe. Ziel ist aber eine möglichst homogene Verteilung der zwei verschiedenen Komponenten des Stoffgemischansatzes, um beispielsweise eine chemische Reaktion zwischen den Bestandteilen einzuleiten und gleichmässig zu fördern, z.B.die nachfolgend beschriebene Polymerisation eines Zwei- oder auch Mehrkomponentengemisches.

Es sind verschiedene Arten von Mischverfahren und Vorrichtungen bekannt. Das Mischen erfolgt dabei beispielsweise durch Rühren, Mengen, Walzen, Kneten, Emulgieren, Suspendieren, Rütteln, Schütteln oder durch Einwirkung von Ultraschall.

Die bekannten Mischverfahren und Mischvorrichtungen wiesen jedoch den Nachteil auf, dass, zunächst aus Applikationsbehälnissen, die einzelnen Komponenten in einem dritten Gefäss angesetzt werden müssen. Beispielsweise ist in der europäischen Patentschrift EP 0261182B1 ein Mischgefäss beschrieben, welches in auseinandermontiertem Zustand zwei Komponenten eines Mischansatzes aufnehmen kann, danach wieder montiert wird und dann zum Mischen unter Vakuumeinsatz bereit ist. Bekanntermassen werden gerade beim Einschütten leicht flüchtiger Komponenten enorme Mengen an Gasen frei, die die am Mischvorgang beteiligten Personen, beispielsweise im Operationssaal, den Dämpfen der Komponente oder dem leicht aufwirbelnden Staub von Pulverkomponenten aussetzt, was zu Allergisierungen führt und alle in der Umgebung des Mischansatzes stehende Apparaturen kontaminiert.Insbesondere kommt es beim Einschütten aus den zu eröffnenden Behältnissen und Verpackungen oft zum Verlust von Bestandteilen der Komponenten, indem diese verschüttet werden oder nicht vollständig aus dem Behältnis entleert werden können. Oft geschieht es in der Hektik der Situation, dass, beispielsweise im Operationssaal, Verpackungsbehältnisse, die für den Ansatz gerichtet sind, umgeschüttet werden und so teure Substanzen zu ersetzen sind, ganz abgesehen davon, dass meist der gesamte Instrumentiertisch neu gerichtet werden muss. Insbesondere aber kommt es beim Einschütten der Komponenten oft zum Aufwirbeln von Pulver bei pulvrigen Komponenten und zum Aufspritzen von Flüssigkeit bei flüssigen Komponenten, so dass das Mischgefäss einzelne Komponentenbestandteile oder unvollständig gemischter Komponentenansätze wandständig aufweist und so ein homogenes Gemisch nicht mehr möglich wird.

Es hat nicht an Versuchen gefehlt, wie die Patentanmeldungen EP A0170120, DE-A-334783 oder US-A-3779371 beweisen, dieses missliche und für alle Beteiligten oft umständliche Verfahren durch reproduzierbare, sichere und geeignete Mischvorrichtungen zu ersetzen. Spezielle Probleme treten vor allem dann auf, wenn ein sog. "Knochenzement" im Operationssaal angesetzt werden soll.

Als Knochenzemente werden meist kalt polymerisierende Zweikomponenten-Kunststoffe verwendet, durch die die Komponenten künstlicher Gelenke im knöchernen Bett verankert werden. Der Knochenzement härtet unmittelbar nach der Applikation im Knochen aus und erreicht durch seine plastischen Eigenschaften ein Eindringen in die Knochenstruktur und so ein sog. "Interlocking" des Knochenzementes im Knochen mit fester Verankerung der Prothesenkomponente in der Knochenzementscheide. Seit vielen Jahren werden als Knochenzemente Polymethylmetacrylate (PMMA) verwendet. Diese bestehen aus einem pulverförmigen Perlpolymerisat,welches in flüssigem Monomer angelöst und schliesslich durch Polymerisation des Monomers in dieses eingebettet wird. Neuerdings werden auch granulathaltige PMMA-Pulver verwandt. In der Mischphase umfliesst das Monomer das kugel- oder granulatförmige Polymerpulver, dabei gibt es zunächst eine Partikelaufschwemmung, in der mehr oder weniger viele Luftblasen mit eingeschlossen werden. Die Partikelgrössen dieser Pulverkomponenten liegen zwischen 1 und 140 µm, manchmal geringfügig oberhalb dieser Grössenordnung. D.h., es handelt sich um griessförmige, in jedem Fall aber auch aufstäubende Pulverkomponenten, die beim Einschütten in ein Mischgefäss an elektrostatisch sich aufladenden Gefässwänden haften, meist doppelverblistert in Papierfolientüten geliefert werden, und erhebliche Probleme bereiten durch Aufstäuben und Haften beim Einschütten in das Mischgefäss. Es bedarf einer besonderen Handfertigkeit und Geschicklichkeit, um solche in Tüten angelieferte Perl- oder Granulatpolymerisate artefaktfrei einzuschütten, ohne die Umgebung zu kontaminieren oder Substanzen zu verlieren. Beim Ansatz solcher Komponenten besteht die Pulverkomponente immer aus einem hochmolekularen Polymerisat des Zweikomponentenwerkstoffes, während die flüssige Komponente immer aus den monomeren Bestandteilen dieser Komponenten besteht. Schon aus diesem Grunde ist es zwingend, die flüssige Komponente vorzulegen, damit das schwerere Polymerpulver in den Flüssigkeitssee absinken und ein Grossteil der im Pulver eingeschlossenen Luftblasen bereits abstreifen kann. Da dem Pulver Starterkomponenten und der Flüssigkeit Initiatoren der Starterkomponente zugefügt sind, beginnt unmittelbar nach Zusammenkommen von Flüssigkeit und Pulver ein sog. "Polymerisationsprozess", der um so gleichmässiger abläuft, je gleichzeitiger die einzelnen Bestandteile aus beiden Komponenten miteinander in Kontakt kommen.

In der Regel wird das Polymerpulver dem Monomer beigegeben und mit einem Spatel in einer Schale vermischt. In der an die Mischphase anschliessenden Verarbeitungsphase wird der Knochenzement, meist von Hand, teilweise auch mit einer Spritze, in das knöcherne Lager eingebracht. Beispielsweise kann man durch Kneten das Gemisch, in einem Zustand, in dem die Komponentenmasse nicht mehr am chirurgischen Handschuh kleben bleibt, in der Form verarbeiten, dass es zu einer Wurst gerollt wird und dann in geeigneter Form mit dem Finger in die Markhöhle des eröffneten Oberschenkelknochens eingestopft wird. Nach Auffüllen des proximalen Endes dieses Knochenrohres, wird dann im Anschluss daran die Prothesenkomponente eingeführt. Allein durch die Verwendung einer Knochenzementspritze können, im Hinblick auf die Zementverankerung im Knochen, wesentlich bessere Ergebnisse als mit der herkömmlichen sog. "Fingerstopfmethode" erzielt werden.

Da die Dauerschwingfestigkeit des Zementes ein wesentliches Kriterium für die Dauerhaltbarkeit der Verankerung ist, hat es nicht an Versuchen gefehlt, die Festigkeit des Knochenzementes dadurch zu erhöhen, dass das Gemisch im Vakuum hergestellt wurde.

Serienuntersuchungen dieser Mischproben haben jedoch gezeigt, dass ein wesentliches Kriterium der Dauerfestigkeit dieser Materialien in der Homogenität der Verteilung der beiden Komponentenanteile untereinander besteht. Diese sog. "Homogenität" des Gemisches ist jedoch ein sehr heikler Vorgang, der beim Ansatz und bereits beim Verpacken der Komponenten berücksichtigt werden muss.

So kommt es beispielsweise beim Vorlegen des monomeren Bestandteiles beider Komponenten in dem Mischgefäss zum Spritzen des Monomers entlang der Wand des Gefässes. Beim danach folgenden Einschütten der Pulverkomponente stäubt das Pulver auf, und die Spritzer der ersten Komponente werden unvollständig mit dem Pulver in Berührung gebracht und damit der Polymerisationsvorgang in all den kleinen Spritzern gestartet. Da die Zusammensetzung ausserhalb des Hauptgemisches anteilmässig nicht mehr korrekt gegeben ist, kommt es in diesen inselförmigen, unvollständig oder gar nicht vermischten Komponentenanteilen zu den unterschiedlichsten Reaktionsergebnissen. Beim anschliessenden Extrudieren eines solchen Gemisches, wie dies beispielsweise in der EP 0261182B1 beschrieben und geschildert ist, kommt es zum Eindrücken dieser inhomogenen Bestandteile in das Gemisch, wo sie meist als inhomogene Inseln im Röntgenbild erkannt werden können.

Oft gehen aber auch beim Ansetzen der Komponentenbestandteile Anteile der einzelnen Komponenten verloren. Es kostet Zeit und zusätzliche Instrumente, um die Verpackungen korrekt zu öffnen und den Ansatz im Mischgefäss herzustellen. Um nun dieses Problem zu lösen, konnten rein zufällig, durch die nachfolgend geschilderte Erfindung, alle im Zusammenhang mit dem Ansatz auftretenden Probleme elegant, einfach und preiswert gelöst werden. Da am Beispiel des Knochenzementes die flüssige Monomerkomponente, die reines Methylmetacrylat-Monomer unter Zusatz von Dimethyl-p-toluidin und als Stabilisator meist eine Substanz wie Hydrochinon enthält, gezeigt werden kann, dass es eine leicht flüchtige Substanz und eine licht-empfindliche Substanz darstellt, die unter UV-Strahlen polymerisiert. Eine solche Glasamphiole kann unter Sonnenbestrahlung sehr langsam zum transparenten PMMA-Acrylat aushärten. Aus diesem Grunde werden meist alle zur Verfügung stehenden Monomerkomponenten eines solchen PMMA Zweikomponentengemisches in dunklen Glasamphiolen geliefert und, sorgfältig verpackt, vor Lichteinstrahlung geschützt und an einem nicht temperaturexponierten Platz aufbewahrt. Die Sicherung des Monomers in einer solchen Glasamphiole bedarf einer sorgfältigen Validierung des Verfahrens, um auch den Gebrauch der Monomerkomponente noch nach Jahren sicherzustellen. Die Monomerkomponente, die als flüchtiger Bestandteil, zumindest in der Zellkultur als toxisch angesehen werden muss, ist schon aus umwelttechnischen Gründen sorgfältig und transportsicher geschützt aufzubewahren, um bei Unfällen eine Kontamination mit dieser leicht flüchtigen Substanz zu verhindern. Die polymere Perlpolymerisat- oder Granulatkomponente wird meist dreifach verpackt in sog. "Aufreissbeuteln" mit Sichtfolie und Papierseite, welche bakteriendicht, aber gasdurchlässig ist, in einer vor Feuchtigkeit schützenden und lichtdichten Aluminiumverpackung verblistert aufbewahrt. In sterilem Zustand sind in dieser Weise sowohl Monomer-, als auch Polymerkomponente über Jahre haltbar steril aufbewahrbar.

Das Auspacken und das Ansetzen aus diesen Verpackungen im Mischgefäss ist aber mit einem gehörigen Aufwand verbunden und bedarf besonderer manueller Geschicklichkeit, um zu einem homogenen Gemisch, ohne Verlust von Komponentenanteilen zu kommen.

In dem US Patent 4,408,690 wird beispielsweise eine Mischvorrichtung beschrieben, bei der zwei Kammern durch ein Diaphragma getrennt sind und über eine Schneidevorrichtung die sich in einer der beiden Kammern befindet und ein Saugröhrchen aufnehmen kann welches das Siegel der Kammer durchstösst und zusammen mit der Schneidevorrichtung die Membran durchstösst und auf diese Weise beide Kammern miteinander verbindet und durch axiales Drehen das Getränkt mit der Komponente der oberen Kammer mischt Bei einem Getränkt spielt es keine Rolle wenn Bestandteile der Kammerverschlüsse in das Mischgefäss fallen, auch geht man davon aus dass das Basisgetränk sich bereits in dem Bechergefäss befindet was bei einem Biomaterial wie es der Knochenzement darstellt, natürlich nicht möglich ist; hier muss das Monomer aus einer chemisch sehr beständigen Glasamphiole zugesetzt werden und das Gemisch muss unter Vakuum homogen vermengt werden können, auch muss unter allen Umständen vermieden werden, dass Bestandteile der Kammerverschlüsse in das Gemisch geraten da sie sonst als Teil des Knochenzementes mit implantiertt werden würden.

Mit der nachfolgend geschilderten Erfindung konnten die mit der Lagerung, Aufbewahrung und Verpackung und, im Zusammenhang mit dem Mischansatz sich ergebenden Probleme elegant gelöst werden.

Ein kugelförmiges Behältnis, welches auf einen zylinderförmigen Becher vakuumdicht aufgesetzt werden kann und welches an beiden Polen durch eine oben geschilderte Papiermembran mit einer mindestens 8 mm breiten geklebten Verbindungsnaht zum Gefässrand verschlossen ist, kann als Aufbewahrungsgefäss für ein Perlpolymerisat oder eine Granulatkomponente oder einem Gemisch aus beidem dienen. Durch die papierförmigen Membranen kann Ethylenoxyd, welches sich besonders eignet, um die Pulverkomponente zu sterilisieren, penetrieren und so für die Keimfreiheit des Behältnisses sorgen. Durch Aufsetzen des Gefässes, welches vorteilhaft an seinem Rand oberhalb des Becherrandes, auf den es vakuumdicht aufgestülpt wird, mit einem Saugstutzen für das Anlegen von Vakuum versehen ist, kann durch eine zweite Gummidichtung eine angeschliffene Kanüle eingestochen werden und, durch Anlegen des Vakuums an den Vakuumsaugstutzen, aus der eröffneten Amphiole vom Boden des Gefässes weg die Monomerkomponente spritzfrei aufgesaugt und im Mischbecher überführt werden. Nach Einfüllen der Monomerkomponente in dieser Weise, bei der es nicht zum Freiwerden von Monomerdämpfen kommt, da alle Dämpfe in der Glasamphiole über die Kanüle durch das Vakuum mit aufgesaugt werden, kann die Kanüle entfernt werden, und die elastische Deformierung des Gummisiegels schliesst das Behältnis für den Mischansatz wiederum vakuumdicht ab. Im Anschluss daran kann durch den Deckel des Behältnisses der Monomerkomponente, nach Abreissen der aufgeklebten Papiermembran, in einem zentral kanülierten Gummisiegel ein Rührstab eingechoben werden, der vorteilhafterweise beim Mischen von PMMA-Knochenzementen mit Teflon beschichtet ist, um ein Anhaften des auspolymerisierenden Knochenzementes zu verhindern. Dieser gerade Rührstab kann durch die Polymerkomponente des Mischansatzes durch die Membran oder den Deckel, der das Gefäss nach unten verschliesst, hindurchgestossen werden, so dass die Membran, entlang von vorgegebenen Perforationslinien aufreisst und die Pulverkomponente sich entlang dem

vorsichtig gedrehten Rührstab in die Monomerkomponente einschüttet. Nachdem in dieser Weise das Perlpolymerisat oder -granulat oder ein Gemisch aus beiden der Pulverkomponente aus dem aufgesetzten Behältnis entfernt worden ist, wird, unter Anlegen eines Vakuums, der Ansatz gemischt und in der in der EP 0261182B1 geschilderten typischen Mischphase unter Vakuum gerührt: In einer ersten, sehr turbulenten Mischphase, werden die beiden Komponenten, die flüssige Monomerkomponente und die pulverförmige Polymerkomponente, kräftig untereinandergerührt, so dass keine freien Pulverbestandteile mehr sichtbar sind. Dieser Mischprozess dauert in der Regel 10 - 15 sec (10 sec Einrühren ohne Vakuum, 15 sec mit Vakuum). In der daran anschliessenden Polymerisationsphase des Gemisches wird in mehr laminaren Schichten gerührt mit der halben Drehzahl (1 - 2 Umdrehungen/sec gegenüber 3 - 4 in der turbulenten Mischphase) und in der sich daran anschliessenden Nachevakuierungsphase über 15 sec werden die durch das Rühren eingebrachten Luftblasen herausgesaugt, und auf diese Weise entsteht ein blasenfreies Gemisch, welches nach Belüftung des Gefässes in der beispielsweise in der EP 0261182B1 beschriebenen Weise extrudiert werden kann, in ein Applikationsgefäss, über das es dann direkt in das Knochenbett eingebracht werden kann. Der gesamte Mischvorgang dauert damit 55 Sekunden.

In einer besonderen Variante des Gefässes kann über einen Stutzen aus einem elastisch deformierbaren Material, in aller Regel Silicon, die Glasamphiole in umgekehrter Weise mit ihrem spitzen schlanken Hals direkt aufgenommen und in der Weise umschlossen werden, dass es in dem Stutzen gebrochen und anschliessend unter Vakuum die Flüssigkeit eingesaugt werden kann. Auf diese Weise wird das Splittern der Amphiole sicher verhindert, und das Monomer kann kontaminationsfrei in das Mischgefäss eingesaugt werden. Die Glasamphiole kann während des Mischvorganges im Stutzen stecken bleiben, sie kann aber auch entfernt werden. Der Stutzen wird sich durch die elastische Deformation des Materials von selbst vakuumdicht verschliessen und hält in jedem Falle den Hals und die Spitze der Amphiole im Stutzengang fest.

Die vorstehend erläuterte Ausführungsform kann als ein "geschlossenes System" bezeichnet werden, da die Bestandteile des Stoffgemisches, wie z.B. des Knochenzementes, abgeschlossen und getrennt voneinander aufbewahrt werden können und während der gesamten Verarbeitung und Mischung bis zum Einfüllen in den Applikationsbehälter nicht mit dem Operateur oder der Umgebung in Berührung kommen. Beide Behälter können aus demselben Materiel bestehen, vorzugsweise ist jedoch der obere Verschluss des Gefässes, welches die Pulverkomponente enthält, aus einem elastischen Material, beispielsweise Silicon, desgleichen die Siegel, die mit der scharf gespitzten Saugkanüle durchstossen werden können. Der Ansaugstutzen für das Vakuum in dem Deckel bildenden Polymergefäss kann entweder aus einem Kunststoff als Saugstutzen in einen Kanal eingeführt werden, oder aber schon als fester Bestandteil dieses Behältnisses in der Konstruktion berücksichtigt sein und aus demselben Material bestehen.

Es kann ein Kunststoff sein, vorzugsweise ein thermoplastischer Kunststoff, wiebeispielsweise ein Polyolefin. Besonders bevorzugt ist die Verwendung von Poly (4-Methyl-1-Penten) oder TPX®. Es kann auch ein Polycarbonat verwandt werden oder im einfachsten Falle ein Polystyrol. Das Monomer, welches im dritten Behälter aufbewahrt wird (Monomerbehälter), wird in aller Regel in einer Glasamphiole aufbewahrt, es kann aber auch aus einem Gefäss aus Teflon oder aus einem mit Teflon beschichteten Gefäss bestehen oder in dem gehämmerten Behälter des Polymers integriert sein.

Die Durchtrittsstutzen bzw. -siegel des Pulverbehältnisses werden vorzugsweise aus einem Silicon hergestellt, es kann aber auch ein Kunststoffgelenk mit Durchlass eingebaut sein, welches die Aufnahme des Rührstabes und die Beweglichkeit des Rührstabes im Behältnis garantiert. Die Erfindung wird nachstehend anhand von Ausführungsbeispielen, die das Mischen, das Aufbewahren und den Ansatz eines Zweikomponenten-Knochenzementes betreffen und anhand der Zeichnungen näher erläutert. Es zeigen:
Abb 01 Eine Vorrichtung zum Aufbewahren der Pulverkomponente und der Monomerkomponente mit einem integrierten Mischgefäss, welchem das Behältnis, welches die Pulverkomponente enthält, vakuumdicht aufgesetzt werden kann.
Abb 02 Dasselbe Gefäss mit eröffneter Amphiole, welche das Monomer enthält das bereits durch die durch das Siegel eingestochene Vakuumkanüle eingesaugt wird.
Abb 03 und 04 Mischvorgang mit Durchstossen einer Papiermembran mit dem Einmischen der Pulverkomponente in die flüssige Komponente und dem Mischen der beiden Komponenten zum homogenen Gemisch.

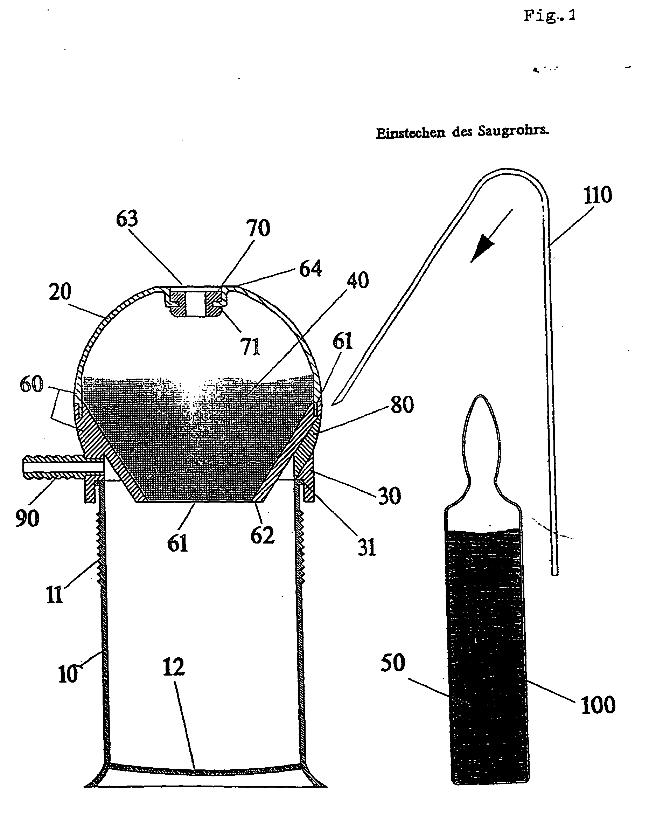

In der Abb 01 ist detailliert ein Behältnis für die Polymerkomponente zu erkennen (Pulverkomponente eines Zweikomponenten-Knochenzementes. Die Abb 01 zeigt ein Behältnis (60), welches aus fertigungstechnischen Gründen aus zwei halbkugelförmigen Kalotten besteht (20 und 30). Diese sind in einer zirkulären Nut ultraschallverschweisst und bestehen aus dem temperaturbeständigen Poly(4)-Methyl-Penten (TPX). Axial sind die Pole des Gefässes mit einer gasdurchlässigen, aber bakteriendichten Membran verschlossen (61,63). Die Papiermembran ist auf einer 8 mm breiten Auflage dicht verklebt (62,64). In dem Behältnis befinden sich 40 g eines Granulat-Perlpolymerisatgemisches eines PMMA mit einem mittleren Molekulargewicht von 500.000. Die obere (proximale) Öffnung ist unterhalb der Papierabdichtung (63) mit einem kanülierten Gummisiegel verschlossen (70), welches in einer Nut (71) vakuumdicht gegenüber dem Behältnis (20) abschliesst. Das Behältnis (60) sitzt vakuumdicht einem planen Becherrand (31) auf und lässt, entlang seiner unteren Kugelkalottenhälfte (30) über einen mit Silicon versiegelten Kanal mit einer geschliffenen Durchstechkanüle den Zutritt zum Bechergefäss (10) frei.. Über einen sich unter dem Kalottenteil (30) des Polymerbehälters (60) befindlichen Saugstutzen (90) kann das Mischgefäss (10) evakuiert werden. Das Mischgefäss wird vorteilhafterweise mit einer Griffleiste (11) versehen, damit ein stabiler Stand durch sicheren Handgriff gewährleistet ist. Der Boden des Mischgefässes ist konkav gestaltet (12), um ein späteres Extrudieren des Gemisches rückstandsfrei zu ermöglichen.

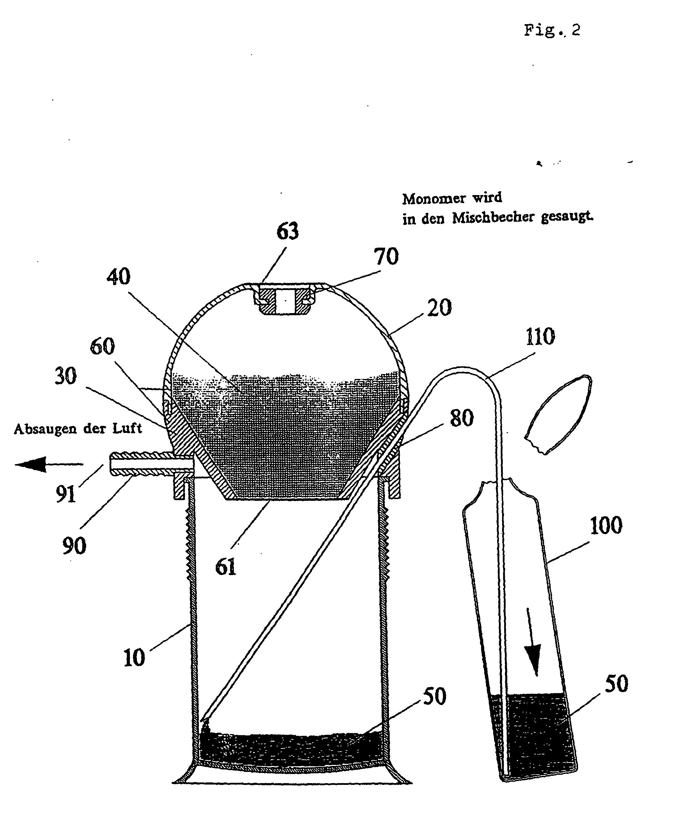

In der Abb 02 ist das Bechergefäß (10) dargestellt mit vakuumdicht aufsitzendem Polymerbehälter (60) und zeigt die bereits durch das Siegel (80) durchgestossenen Saugkanüle (110), ein gefädelt in das Monomergefäß (100), aus dem bereits das Monomer (50) zu über der Hälfte in den Becher (10) durch Anlegen eines Vakuums (91) am Vakuumsaugstutzen (90) herübergesaugt worden ist. Das Polymergefäss (60) ist mit beiden Membranen (61,63) und dem Gummisiegel (70) versehen und verschlossen.

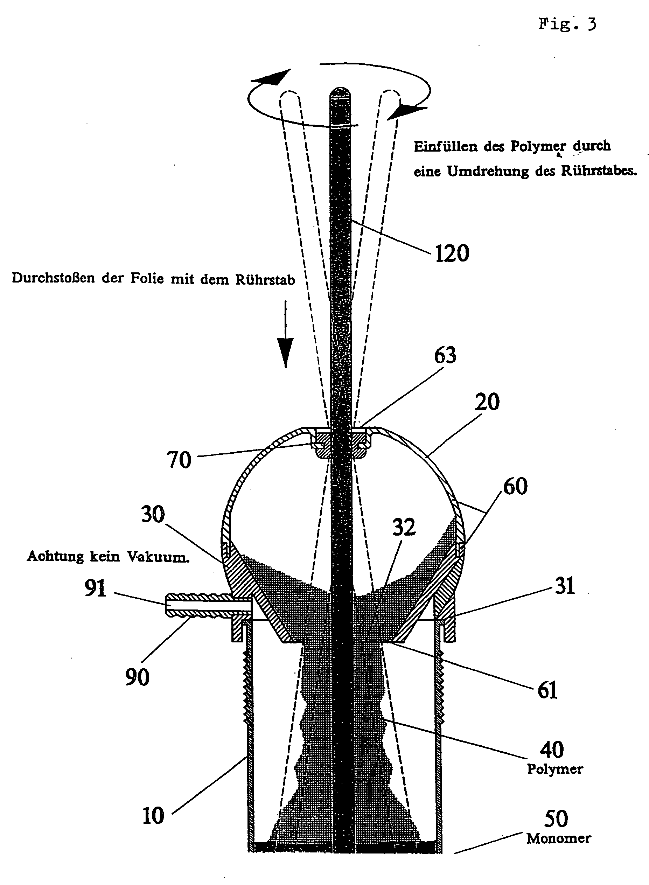

Die Abb 03 zeigt das Polymergefäss über die Membranen (63,61), durch den geraden teflonbeschichteten Rührstab (120) eröffnet, aufsitzend auf dem Bechergefäss (10) mit Vakuumabdichtung (31) und dem Vakuumstutzen (90), angeschlossen an das Vakuum (91) mit herausfallendem Polymerpulver (40) in den Monomersee (50) im Becher (10) aus dem Polymerbehältnis (60), welches aus den beiden Kalotten (30,20) besteht. Der Rührstab (120) kann über die Öffnung (32) alle Punkte des Bodens im Bechergefäss (10) erreichen.

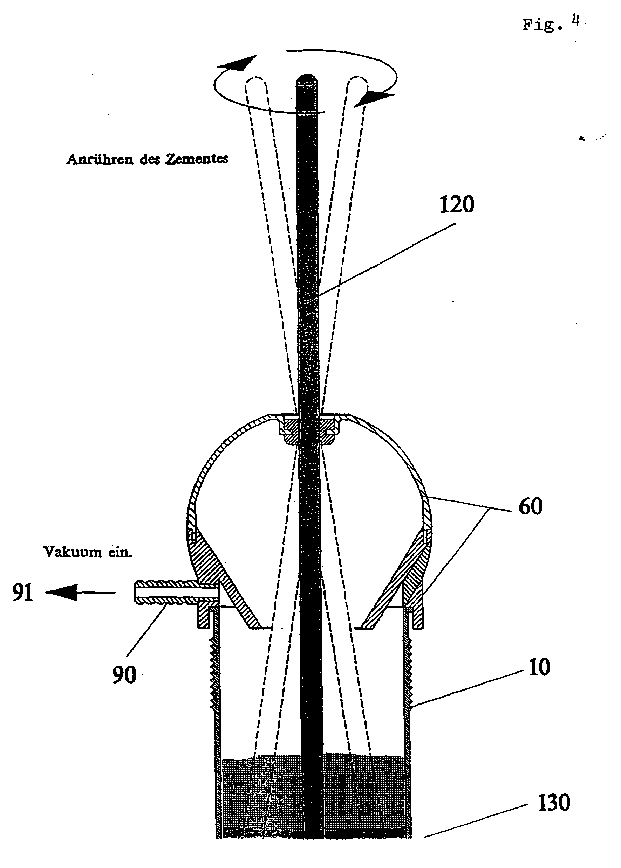

In der Abb 04 ist das Pulverbehältnis (60) mit dem durchtretenden Rührstab (120), vakuumdicht aufsitzend auf dem Becher (10), dargestellt mit dem Inhalt des homogenen Gemisches aus Polymer und Monomer (130), unter angelegtem Vakuum (91) über den Vakuumsaugstutzen (90).

Das in dieser Weise hergestellte homogene Gemisch wird nach Abschalten des Vakuums mit dem am Becherrand gehaltenen Rührstab in der Weise weiter bearbeitet, indem der aufgesetzte Polymerbehälter (60) mit dem Rührstab (120) zusammen entfernt wird, indem der Rührstab am Becherrand entlang hochgeführt wird, damit ein Eintropfen vom am Rührstab herabfliessendem Knochenzement verhindert wird. Im Anschluss daran kann der Knochenzement aus dem nun offenstehenden Gefäss (10) mit Hilfe einer Extrudierlamelle in ein Applikationsgefäss, z.B. eine zylinderförmige Kartusche, mit einem an die Eröffnung des Knochens angepassten Maul extrudiert werden.

In einer besonderen Ausführungsform des Einlassstutzens aus Silikon (70) ist der durchführende Kanal in der Weise schräg ausgebildet, daß der Rührstab in Ruheposition immer in der Ecke des Bechergefässes (10) gehalten wird und so ein Herausziehen des Rührstabes entlang des Becherrandes erzwungen wird.

## Patentansprüche

1. Vorrichtung zum Abpacken und Mischen eines aus mindestens zwei Bestandteilen bestehenden Stoffgemischansatzes, einer Pulverkomponente und einer Flüssigkeit, bestehend aus einem Behältnis (20) und einem Mischgefäss (10), dessen Behältnis an mindestens zwei Polen temporär verschliessbare Öffnungen aufweist und durch die folgenden Merkmale **gekennzeichnet** ist:
das Behältnis (20) kann vakuumdicht dem evakuierbaren Mischgefäss (10) aufgesetzt und von einer Kanüle (110) durchstossen werden, nach deren Entfernung sich die Öffnung im Siegel (80) elastisch vakuumdicht verschliesst, und ist so beschaffen, dass durch die eine, durch eine von aussen entfernbare Membran (63), verschlossene Öffnung ein Rührstab (120) vakuumdicht passieren und die, von einer anderen Membran (61), verschlossene Öffnung durchstossen kann, wobei die Öffnung so beschaffen ist, dass der Rührstab (120) den Boden (12) des Mischgefässes in jedem Punkt berühren kann.

2. Vorrichtung nach Anspruch 1, so beschaffen, dass die Öffnungen an beiden Polen angeordnet sind und dass eine der Öffnungen in der Weise verschlossen ist, dass der Verschluss von aussen entfernt werden kann.

3. Vorrichtung nach den Ansprüchen 1 und 2, so beschaffen, dass die unter Anspruch 2 beschriebene Öffnung unter dem Verschluss einen elastisch deformierbaren Einsatz oder ein Kugelgelenk aufweist, welche(s)r vakuumdicht von einem Rührstab passiert werden kann.

4. Vorrichtung nach den Ansprüchen 1 - 3, so beschaffen, dass die zweite Öffnung mit einer keimdichten Membran oder einem keimdichten Deckel verschlossen ist, die (der) durch einen Rührstab durchstossen werden kann.

5. Vorrichtung nach den Ansprüchen 1 - 4, so beschaffen, dass die unter Anspruch 4 beschriebene Membran nicht abreisst, sondern entlang vorgegebener Sollrisslinien aufreisst.

6. Vorrichtung nach den Ansprüchen 1- 5, so beschaffen, dass die Membran der Ansprüche 4 und 5 physikalisch und/oder chemisch eröffnet werden kann.

7. Vorrichtung nach den Ansprüchen 4 - 6, so beschaffen, dass die Membran oder der Deckel durch Unterdruck aufreisst bzw. geöffnet wird.

8. Vorrichtung nach den Ansprüchen 1 - 7, derart, dass sie vakuumdicht einem Mischgefäss aufgesetzt werden kann, welches evakuierbar ist.

9. Vorrichtung nach den Ansprüchen 1 - 8, so beschaffen, dass das Behältnis eine weitere Öffnung aufweist, an der ein Vakuum angelegt werden kann, welches nicht auf die Kammer des Behältnisses wirkt, sondern dazu dient, dass das zweite Behältnis, auf das das Behältnis der Ansprüche 1 - 8 vakuumdicht aufgesetzt werden kann, evakuiert werden kann.

10. Vorrichtung nach den Ansprüchen 1 - 9, der Art, dass die Membran oder der Deckel an beiden Öffnungen bakteriendicht, aber gasdurchlässig sind und der Inhalt mit Ethylenoxyd sterilisiert werden kann.

11. Vorrichtung nach den Ansprüchen 1 - 10, so beschaffen, dass das Behältnis, an einem Ende verschlossen, dem zweiten Behältnis vakuumdicht aufgesetzt werden kann.

12. Vorrichtung nach den Ansprüchen 1 - 11, so beschaffen, dass das Behältnis über die Vorrichtung des Anspruches 1 - 9 und/oder durch eine Öffnung im Behältnis selbst evakuiert werden kann.

13. Vorrichtung nach den Ansprüchen 1 - 12, so beschaffen, dass die zweite Öffnung des Behältnisses aus den Ansprüchen 1 - 9 so gross ist, dass ein gerader Rührstab den Boden des Behältnisses der Ansprüche 11 und 12 in jedem Punkt berühren kann.

14. Vorrichtung nach den Ansprüchen 1 - 13, so beschaffen, dass es von einer Kanüle durchstossen werden kann, ohne dass die Kammer des Behältnisses der Ansprüche 1 - 9 eröffnet wird und dass die Kanüle entfernt werden kann und sich die Öffnung von selbst elastisch verschliesst und vakuumdicht bleibt.

15. Vorrichtung nach den Ansprüchen 1 - 14, so beschaffen, dass das Stoffgemisch einen Knochenzement auf PMMA Basis darstellt, dessen Pulverkomponente ein PMMA oder ein verwandtes Polymer bildet und dessen Flüssigkeit aus Methylmetacrylat oder einer dieser nahestehenden chemischen Verbindung besteht.

16. Vorrichtung nach den Ansprüchen 1 - 15, so beschaffen, dass das Behältnis gekammert ist und Polymer und Monomer enthält.

17. Vorrichtung nach den Ansprüchen 1 - 16, so beschaffen, dass beide Kammern physikalisch und/oder chemisch zum Mischgefäss zu öffnen sind und/oder von außen direkt oder indirekt geöffnet werden können.

18. Vorrichtung nach Anspruch 17, so beschaffen, dass das Monomer, d.h., die flüssige Komponente, zuerst in das Mischgefäss entleert werden kann.

## Claims

1. A device for packaging and mixing a mixture comprising at least two constituents, a powder constituent and a liquid constituent, comprising a container (20) and a mixing vessel (10), whose container is provided with temporarily closeable openings at least at two poles and is
**characterized through the following features:**
the container (20) can be put onto the mixing vessel (10) in a vacuum tight manner and can be pierced by a cannula (110), after whose removal the opening in the seal (80) closes elastically and vacuum tight, and it is provided so that through the opening closed by one externally removable membrane (63) a stirring rod (120) can pass in a vacuum tight manner and pierce the opening, closed by another membrane (61), wherein the opening is provided in a way that the stirring rod (120) can reach the bottom (12) of the mixing vessel in any spot.

2. A device according to claim 1, **so provided, that**
the openings are located on both poles and one opening is closed in a manner, so that the closure can be removed from the outside.

3. A device according to claims 1 and 2, **so provided, that**
the opening described in claim 2 has an elastic deformable insert or a ball joint under the closure, which can be passed by a stirring rod in a vacuum tight manner.

4. A device according to claims 1 - 3, **so provided, that**
the second opening is closed by a germ proof membrane or by a germ proof lid, which can be pierced by a stirring rod.

5. A device according to claims 1 - 4, **so provided, that**
the membrane described in claim 4 does not tear off, but tears open along predetermined tear lines.

6. A device according to claims 1 - 5, **so provided, that**
the membrane according to claims 4 and 5 can be opened physically and / or chemically.

7. A device according to claims 4 - 6, **so provided, that**
the membrane or the lid tears open, or is opened through low pressure.

8. A device according to claims 1 - 7, **so provided, that**
it can be put on top of a mixing vessel in a vacuum tight manner, which can be evacuated.

9. A device according to claims 1 - 8, **so provided, that**
the container has another opening, where a vacuum can be applied, which does not affect the chamber of the container, but provides for the second container, which can be put on top of the container according to claims 1 - 8 in a vacuum tight manner, to be able to be evacuated.

10. A device according to claims 1 - 9, **so provided, that**
the membrane or the lid on both openings are germ tight, but gas permeable and the content can be sterilized with ethylenoxide.

11. A device according to claims 1 - 10, **so provided, that**
the container closed on one end, can be put on top of the second container in a vacuum tight manner.

12. A device according to claims 1 - 11, **so provided, that**
the container can be evacuated through the device according to claims 1 - 9
and / or through an opening in the container itself.

13. A device according to claims 1 - 12, **so provided, that**
the second opening of the container according to claims 1 - 9 is large enough, so a straight stirring rod can touch the bottom of the container according to claims 11 and 12 in any spot.

14. A device according to claims 1 - 13, **so provided, that**
it can be pierced by a cannula, without opening the chamber of the container according to claims 1 - 9, and the hypodermic needle being removable and the opening self sealing elastically and remaining vacuum tight.

15. A device according to claims 1 - 14, **so provided, that**
the mixture is a PMMA based bone cement, whose powder constituent is a PMMA or a related polymer and whose fluid is methylmetacrylat or a related chemical compound.

16. A device according to claims 1 - 15, **so provided, that**
the container is chambered and contains polymer and monomer.

17. A device according to claims 1 - 16, **so provided, that**
both chambers can be physically and / or mechanically opened towards the mixing vessel and / or can be opened from the outside directly / or indirectly

18. A device according to claim 17, **so provided, that**
the monomer, this means the liquid component, can be emptied first into the mixing vessel.

## Revendications

1. Dispositif pour empaqueter et mélanger une préparation ou composition constituée d'au moins deux composants, un composant en poudre et un liquide, constitué d'un récipient (20) et d'une cuve de mélange (10), lequel récipient présente au moins en deux pôles des ouvertures pouvant se fermer temporairement, et qui est **caractérisé par** les caractéristiques suivantes :
le récipient (20) peut être placé sur la cuve évacuable de mélange (10) de façon hermétique ou étanche au vide et être transpercé par une canule (110), l'ouverture du bouchon (80) se refermant de façon élastique et hermétique ou étanche au vide après le retrait de celle-ci, et est fait de telle façon qu'un bâtonnet agitateur (120) peut passer de façon hermétique ou étanche au vide à travers une ouverture fermée par une membrane amovible de l'extérieur (63) et peut transpercer l'ouverture fermée par une autre membrane (61), l'ouverture étant prévue de telle façon que le bâtonnet agitateur (120) peut toucher le fond (12) de la cuve de mélange en tout point.

2. Dispositif selon la revendication 1, **caractérisé en ce que** les ouvertures sont disposées aux deux pôles et **en ce que** l'une des ouvertures est fermée de façon à ce que la fermeture puisse être retirée de l'extérieur.

3. Dispositif selon les revendications 1 et 2, **caractérisé en ce que** l'ouverture décrite dans la revendication 2 présente un embout élastique déformable ou une articulation orbiculaire sous la fermeture, qui peut être traversé(e) de façon hermétique ou étanche au vide par un bâtonnet agitateur.

4. Dispositif selon les revendications 1-3, **caractérisé en ce que** la deuxième ouverture est fermée par une membrane imperméable aux germes ou un couvercle imperméable aux germes, qui peut être traversé(e) par un bâtonnet agitateur.

5. Dispositif selon les revendications 1-4, **caractérisé en ce que** la membrane décrite à la revendication 4 ne s'arrache pas mais se déchire en suivant un tracé prédéfini.

6. Dispositif selon les revendications 1-5, **caractérisé en ce que** la membrane selon les revendications 4 et 5 peut être ouverte de façon physique et/ou chimique.

7. Dispositif selon les revendications 4-6, **caractérisé en ce que** la membrane ou le couvercle peuvent être déchirés ou ouverts sous l'effet d'une dépression.

8. Dispositif selon les revendications 1-7, **caractérisé en ce qu'**il peut être placé de façon hermétique ou étanche au vide au-dessus d'une cuve de mélange qui peut être évacuée.

9. Dispositif selon les revendications 1-8, **caractérisé en ce que** le récipient présente une autre ouverture par laquelle le vide peut être fait, qui n'agit pas sur la chambre du récipient, mais sert à évacuer le deuxième récipient, sur lequel le récipient des revendications 1-8 peut être placé de façon étanche au vide.

10. Dispositif selon les revendications 1-9, de nature à ce que la membrane ou le couvercle soit imperméable ou étanche aux bactéries mais perméable aux gaz au niveau des deux ouvertures et le contenu puisse être stérilisé avec de l'oxyde d'éthylène.

11. Dispositif selon les revendications 1-10, **caractérisé en ce que** le récipient, fermé à une extrémité, peut être placé de façon hermétique ou étanche au vide au-dessus du deuxième récipient.

12. Dispositif selon les revendications 1-11, **caractérisé en ce que** le récipient peut être évacué par le dispositif des revendications 1-9 et/ou par une ouverture dans le récipient lui-même.

13. Dispositif selon les revendications 1-12, **caractérisé en ce que** la deuxième ouverture du récipient selon les revendications 1-9 est si grande qu'un bâtonnet agitateur droit ou rectiligne peut toucher le fond du récipient selon les revendications 11 et 12 en tout point.

14. Dispositif selon les revendications 1-13, **caractérisé en ce qu'**il peut être transpercé par une canule, sans que la chambre du récipient selon les revendications 1-9 ne soit ouverte, que la canule peut être retirée et que l'ouverture se referme d'elle-même de façon élastique et reste hermétique ou étanche au vide.

15. Dispositif selon les revendications 1-14, **caractérisé en ce que** le mélange représente un ciment osseux à base de PMMA, dont le composant en poudre forme du PMMA ou un polymère apparenté et le liquide est constitué de méthacrylate de méthyle ou d'un composé chimique qui lui est proche.

16. Dispositif selon les revendications 1-15, **caractérisé en ce que** le récipient est compartimenté et contient du polymère et du monomère.

17. Dispositif selon les revendications 1-16, **caractérisé en ce que** les deux chambres s'ouvrent physiquement et/ou chimiquement sur la cuve de mélange et/ou peuvent être ouverts de l'extérieur directement ou indirectement.

18. Dispositif selon la revendication 17, **caractérisé en ce que** le monomère, c'est-à-dire le composant liquide, peut être déversé en premier dans la cuve de mélange.
